# EUROPEAN PATENT SPECIFICATION

(11) **EP 0 998 311 B1**
(45) Date of publication and mention of the grant of the patent: **26.11.2003**
(21) Application number: 99923189.7
(22) Date of filing: 19.05.1999
(51) Int. Cl.: A61L 15/32, A61P 17/02

(54) **HEMOSTATIC SANDWICH BANDAGE COMPRISING A THROMBIN LAYER BETWEEN TWO FIBRINOGEN LAYERS**
BLUTSTILLENDE MEHRSCHICHTIGE BANDAGE, DIE EINE THROMBINSCHICHT ZWISCHEN ZWEI FIBRINOGENSCHICHTEN ENTHÄLT
BANDAGE HEMOSTATIQUE MULTICOUCHES COMPRENANT UNE COUCHE DE THROMBINE ENTRE DEUX COUCHES DE FIBRINOGENE

(30) Priority: 19.05.1998 US 85931 P
(43) Date of publication of application: 10.05.2000
(73) Proprietor: AMERICAN NATIONAL RED CROSS, Washington, DC 20006 (US)
(72) Inventor: MACPHEE, Martin, J., Gaithersburg, MD 20886 (US); DROHAN, William, N., Springfield, VA 22156 (US); BEALL, Dawson, Germantown, MD 20876 (US); FRIEDMAN, Stanley, A., Burtonsville, MD 20866 (US); TUTHILL, David, Frederick, MD 21701 (US); BAYER, Vladislav, Gaithersburg, MD 20878 (US)
(74) Representative: Ritter, Stephen David
(86) International application number: US9910952
(87) International publication number: WO99059647

(56) References cited:
- EP-A- 0 090 997
- WO-A-97/28832
- US-A- 4 606 337
- US-A- 5 702 715
- HOLCOMB J.B. ET AL.: "Implications of new dry fibrin sealant technology for trauma surgery" SURGICAL CLINICS OF NORTH AMERICA, vol. 77, no. 4, 1997, pages 943-950, XP002113927

## Description

### CROSS-REFERENCE TO RELATED APPLICATIONS

This application is the § 371 U.S. National Phase Entry of International Application No. PCT/US99/10952, filed May 19, 1998 and published under PCT Article 21(2) in English, which claims priority to U.S. Provisional Application No. 60/085,931, filed May 19,1998.

### I. FIELD OF THE INVENTION

The present invention relates to a hemostatic sandwich bandage which comprises a plurality of layers that contain resorbable materials and/or coagulation proteins. The inventive hemostatic sandwich bandage is useful for the treatment of wounded tissue.

### II. BACKGROUND OF THE IMVENTION

The control of hemorrhage (bleeding) is a critical step in first aid and field trauma care. Unfortunately, the materials and methods available to stop bleeding in prehospital care (gauze dressings, direct pressure. and tourniquets) have not changed greatly in the past 2000 years. L. Zimmerman *el al., Great Ideas in the History of Surgery* (San Francisco. Calif: Norman Publishing; 1993), 31. Even in good hands they are not uniformly effective, and the occurrence of excessive bleeding or fatal hemorrhage from an accessible site is not uncommon. J.M. Rocko *et al., J. Trauma 22*:635 (1982).

Mortality data from Vietnam indicates that 10% of combat deaths were due to uncontrolled extremity hemorrhage. *SAS*/*STAT Users Guide*, 4th ed. (Cary, NC: SAS Institute Inc; 1990). Up to one third of the deaths from exsanguination during the Vietnam War could have been prevented by the use of effective field hemorrhage control methods. *SAS*/*STAT Users Guide*, 4th ed. (Cary, NC: SAS Institute Inc: 1990).

Although civilian trauma mortality statistics do not provide exact numbers for prehospital deaths from extremity hemorrhage, case and anecdotal reports indicate similar occurrences. J.M. Rocko *et al., J. Trauma 22*:635 (1982). These data suggest that a substantial increase in survival can be effected by the prehospital use of a simple and effective method of hemorrhage control.

Liquid fibrin sealants have been used for years as an operating room adjunct for hemorrhage control. J.L. Garza *et al., J. Trauma 30*:512-513 (1990); H.B. Kram *et al., J. Trauma 30*:97-101 (1990); M.G. Ochsner *et al., J. Trauma 30*:884-887 (1990): T.L. Matthew *et al., Ann. Thorac. Surg. 50*:40-44 (1990); H. Jakob *et al., J. Vasc. Surg., 1*:171-180 (1984). The first mention of tissue glue used for hemostasis dates back to 1909. *Current Trends in Surgical Tissue Adhesives: Proceedings of the First International Symposium on Surgical Adhesives,* M.J. MacPhee *et al*., eds. (Lancaster. Pa: Technomic Publishing Co; 1995). The widespread use of fibrinogen and thrombin was common in the last year of World War II, but was abandoned because of the transmission of hepatitis. D.B. Kendrick. *Blood Program in WW II* (Washington. DC: Office of the Surgeon General, Department of Army; 1989), 363-368.

Currently, single donor fibrin sealants are widely used clinically, not only for hemorrhage control but in various surgical situations. W.D. Spotnitz. *Thromb. Haemost. 74*:482-485 (1995); R. Lerner *et al., J. Surg. Res. 48*:165-181 (1990). Even more extensive use is limited by the strict requirements for temperature control. availability of thawed blood components. and the need for mixing of components. Additional problems with the standard fibrin sealants stem from the transfusion risk of human cryoprecipitate (E.M. Soland *et al., JAMA 274*:1368-1373 (1995)), the low and variable amounts of fibrinogen in the cryoprecipitate (10-30 mg) (P.M. Ness *et al., JAMA 241*:1690-1691 (1979)), hypotensive responses to bovine thrombin (R. Berguer *et al., J. Trauma 31*:408-411 (1991)) and antibody responses to bovine thrombin (S.J. Rapaport *et al*., *Am. J. Clin. Puthol. 97*:84-91 (1992)).

The American Red Cross and others have developed plasma protein purification methods that seem to eliminate the hepatitis risk. R.F. Reiss *et al., Trans. Med. Rev. 10*:85-92 (1996). These products are presently being considered for approval by the Food and Drug Administration.

A dry fibrinogen-thrombin dressing (TACHOCOMB, Hafslund Nycomed Pharma, Linz, Austria) is also available for operating room use in many European countries. U. Schiele *et al., Clin. Materials 9*:169-177 (1992). Present formulations of this dressing use bovine thrombin. While this fibrinogen-thrombin dressing requires no premixing and is easy to use, its utility for field applications is limited by a requirement for storage at 4°C and the necessity for prewetting with saline solution prior to application to the wound.

US 4,606,337 mentions a three-layered resorptive sheet in which fibrinogen is contained in a centrally disposed layer, with thrombin being contained in at least one of the two outer layers.

US 5,702,715 refers to a two layered bandage of fibrinogen and thrombin.

### III. SUMMARY OF THE INVENTION

It is therefore an object of the present invention to provide a hemostatic sandwich bandage that can be used for wound healing. Other objects, features and advantages of the present invention will be set forth in the detailed description of preferred embodiments that follows, and in part will be apparent from the description or may be learned by practice of the invention. These objects and advantages of the invention will be realized and attained by the compositions and methods particularly pointed out in the written description and claims hereof.

In accordance with these and other objects, a first embodiment of the present invention is directed to a hemostatic sandwich bandage for treating wounded tissue in a patient which comprises: (i) a first fibrinogen layer; (ii) a thrombin layer adjacent to the first fibrinogen layer; and (iii) a second fibrinogen layer adjacent to the thrombin layer.

A second embodiment of the present invention is directed to a hemostatic sandwich bandage for treating wounded tissue in a patient which comprises: (i) a resorbable material layer; (ii) a first fibrinogen layer adjacent to the resorbable material layer; (iii) a thrombin layer adjacent to the first fibrinogen layer; and (iv) a second fibrinogen layer adjacent to the thrombin layer.

A third embodiment of the present invention is directed to a hemostatic sandwich bandage for treating wounded tissue in a patient which comprises: (i) a first fibrinogen layer; (ii) a resorbable material layer adjacent to the first fibrinogen layer; (iii) a thrombin layer adjacent to the resorbable material layer; and (iv) a second fibrinogen layer adjacent to the thrombin layer.

Each layer of the inventive hemostatic bandages may also optionally contain one or more suitable fillers, binding agents and/or solubilizing agents. In addition, each of the inventive hemostatic bandages may also optionally further comprise a release layer which contains a release agent and/or a backing material.

A sixth embodiment of the present invention is directed to hemostatic sandwich bandages as indicated above for use in a method of treating wounded tissue.

It is to be understood that both the foregoing general description and the following detailed description are exemplary and explanatory only and are intended to provide further explanation of the invention as claimed.

### IV. DETAILED DESCRIPTION OF THE PREFERRED EMBODIMENTS

### A. DEFINITIONS

Unless defined otherwise, all technical and scientific terms used herein have the same meaning as is commonly understood by one of skill in the art to which this invention belongs. All patents and publications mentioned herein are incorporated by reference.

"Patient" as used herein refers to human or animal individuals in need of medical care and/or treatment.

"Wound" as used herein refers to any damage to any tissue of a patient that results in the loss of blood from the circulatory system. The tissue may be an internal tissue, such as an organ or blood vessel, or an external tissue, such as the skin. The loss of blood may be internal, such as from a ruptured organ, or external, such as from a laceration. A wound may be in a soft tissue, such as an organ, or in hard tissue, such as bone. The damage may have been caused by any agent or source, including traumatic injury, infection or surgical intervention.

"Resorbable material" as used herein refers to a material that is broken down spontaneously and/or by the mammalian body into components which are consumed or eliminated in such a manner as not to interfere significantly with wound healing and/or tissue regeneration, and without causing any significant metabolic disturbance.

"Stability" as used herein refers to the retention of those characteristics of a material that determine activity and/or function.

"Binding agent" as used herein refers to a compound or mixture of compounds that improves the adherence of one layer of the inventive hemostatic sandwich bandage to one or more different layers and/or the adherence of the components of a given layer to other components of that layer.

"Solubilizing agent" as used herein refers to a compound or mixture of compounds that improves the dissolution of a protein or proteins in aqueous solvent.

"Filler" as used herein refers to a compound or mixture of compounds that provide bulk and/or porosity to one or more layers of the inventive hemostatic sandwich bandages.

"Release agent" as used herein refers to a compound or mixture of compounds that facilitates removal of an inventive hemostatic sandwich bandage from a manufacturing mold.

"Foaming agent" as used herein refers to a compound or mixture of compounds that produces gas when hydrated under suitable conditions.

### B. PREFERRED EMBODIMENTS

A first preferred embodiment of the present invention is directed to a hemostatic sandwich bandage for treating wounded tissue in a patient which comprises:(i) a first fibrinogen layer; (ii) a thrombin layer adjacent to the first fibrinogen layer; and (iii) a second fibrinogen layer adjacent to the thrombin layer.

A second embodiment of the present invention is directed to a hemostatic sandwich bandage for treating wounded tissue in a patient which comprises: (i) a resorbable material layer; (ii) a first fibrinogen layer adjacent to the resorbable material layer; (iii) a thrombin layer adjacent to the first fibrinogen layer; and (iv) a second fibrinogen layer adjacent to the thrombin layer.

A third embodiment of the present invention is directed to a hemostatic sandwich bandage for treating wounded tissue in a patient which comprises: (i) a first fibrinogen layer; (ii) a resorbable material layer adjacent to the first fibrinogen layer; (iii) a thrombin layer adjacent to the resorbable material layer; and (iv) a second fibrinogen layer adjacent to the thrombin layer.

Each layer of the inventive hemostatic sandwich bandages may also optionally contain one or more suitable fillers, such as sucrose.

Each layer of the inventive hemostatic sandwich bandages may also optionally contain one or more suitable binding agents, such as sucrose.

Each layer of the inventive hemostatic sandwich bandages may also optionally contain one or more suitable solubilizing agents such as sucrose.

Each layer of the inventive hemostatic sandwich bandages may also optionally contain one or more suitable foaming agents, such as a mixture of citric acid and sodium bicarbonate.

Each of the inventive hemostatic sandwich bandages may also optionally further comprise a release layer which contains a release agent. A preferred release agent is sucrose.

Each of the inventive hemostatic sandwich bandages may also further comprise a backing material on the side of the bandage opposite the wound-facing side. The backing material may be affixed with a physiologically-acceptable adhesive or may be self-adhering (*e.g.* by having a sufficient surface static charge). The backing material may be a resorbable material or a non-resorbable material, such as a silicone patch or plastic. Preferably, the backing material is a resorbable material.

The fibrinogen employed in the inventive hemostatic sandwich bandage is preferably Topical Fibrinogen Complex (TFC), but any suitable fibrinogen. or derivative or metabolite thereof (such as fibrinopeptide A and fibrinopeptide B), may be employed as desired. A specific fibrinogen (or fibrinogen-containing composition) for a particular application may be selected empirically by one skilled in the art. The fibrinogen may also contain Factor XIII.

TFC is a mixture of human plasma proteins which have been purified to an appropriate level and virally inactivated. A preferred aqueous solution of TFC contains 100-130 mg/mL total protein, of which at least 80% is fibrinogen. Other constituents of TFC include albumin (generally about 5-25 mg/mL); plasminogen (generally about 5 mg/mL): Factor XIII (generally about 10-40 Units/mL); and polysorbate 80 (no more than 3%). The pH of TFC is generally in the range of 7.1-7.5. Suitable TFC may also contain fibronectin.

The thrombin employed in the inventive hemostatic bandage is preferably a lyophilized mixture of human plasma proteins which have been purified to an appropriate level and virally inactivated. A preferred aqueous solution of thrombin contains thrombin at a potency of about 300 ± 50 International Units/mL. Other constituents include albumin (generally about 5 mg/mL) and glycine (generally about 0.3 M ± 0.05M). The pH of the preferred thrombin is generally in the range of 6.5-7.1.

Additionally, in each of the embodiments of the present invention, thrombin may be replaced by any of the agents known by those skilled in the art to be activators of fibrin formation. Illustrative examples of such agents are snake venoms. A specific activator of fibrin formation for a particular application may be selected empirically by one skilled in the art.

Any suitable resorbable material known to those skilled in the art may be employed in the present invention. For example, the resorbable material may be a proteinaceous substance, such as silk, fibrin, keratin, collagen and/or gelatin, or a carbohydrate substances, such as alginates, chitin, cellulose, proteoglycans (*e.g.* poly-N-acetyl glucosamine), glycolic acid polymers. lactic acid polymers, or glycolic acid/lactic acid co-polymers. Specific resorbable material(s) for a particular application may be selected empirically by those skilled in the art.

Preferably, the resorbable material is a carbohydrate substance. Illustrative examples of particularly preferred resorbable materials are sold under the tradenames VICRYL and

### DEXXON.

The various layers of the inventive hemostatic sandwich bandage may be affixed to one another by any suitable means known and available to those skilled in the art. Preferably, the fibrinogen layer(s) and/or the thrombin layer(s) is (are) applied as a series of quick-frozen aqueous solution layers and subsequently lyophilized or freeze-dried.

In a particularly preferred embodiment of the present invention, when the inventive bandages are prepared using a mold. a release agent, such as sucrose, is applied to the mold before the first layer of the bandage being prepared. In such embodiments, the inventive hemostatic sandwich bandage further comprises a release layer, which contains said release agent, adjacent to the (i) layer and on the opposite side from the (ii) layer.

Alternatively, a physiologically-acceptable adhesive may applied to the resorbable material and/or the backing material (when present) and the fibrinogen layer(s) and/or the thrombin layer(s) subsequently affixed thereto.

In one embodiment of the inventive sandwich bandage, the physiologically-acceptable adhesive has a shear strength and/or structure such that the resorbable material and/or backing material can be separated from the fibrinogen layer and/or the thrombin layer after application of the bandage to wounded tissue. In another embodiment, the physiologically-acceptable adhesive has a shear strength such that the resorbable material and/or backing material cannot be separated from the fibrinogen layer and/or the thrombin layer after application of the bandage to wounded tissue.

Suitable fibrinogen and thrombin may be obtained from human or mammalian plasma by any of the purification methods known and available to those skilled in the art: from supernatants or pastes of recombinant tissue culture, viruses, yeast, bacteria, or the like that contain a gene that expresses a human or mammalian plasma protein which has been introduced according to standard recombinant DNA techniques: or from the fluids (*e.g.* blood, milk, lymph, urine or the like) of transgenic animals that contain a gene that expresses human fibrinogen and/or human thrombin which has been introduced according to standard transgenic techniques.

As a general proposition, the purity of the fibrinogen and/or the thrombin for use in the inventive hemostatic sandwich bandage will preferably be an appropriate purity known to one of ordinary skill in the relevant art to lead to the optimal efficacy and stability of the protein. Preferably, the fibrinogen and/or the thrombin has been subjected to multiple chromatographic purfication steps, such as affinity chromatography and preferably immunoaffinity chromatography, to remove substances which cause fragmentation, activation and/or degradation of the fibrinogen and/or the thrombin during manufacture, storage and/or use. Illustrative examples of such substances that are preferably removed by purification include protein contaminants, such as inter-alpha trypsin inhibitor and pre-alpha trypsin inhibitor; non-protein contaminants, such as lipids: and mixtures of protein and non-protein contaminants, such as lipoproteins.

The concentration of the fibrinogen and/or the thrombin employed in the inventive hemostatic sandwich bandage is also preferably selected to optimize both the efficacy and stability thereof, as may be determined empirically by one skilled in the relevant art. During use of the inventive hemostatic sandwich bandage, the fibrinogen and the thrombin are preferably activated at the time the bandage is applied to the wounded tissue by the endogenous fluids of the patient escaping from the hemorrhaging wound. Alternatively, in situations where fluid loss from the wounded tissue is insufficient to provide adequate hydration of the protein layers, the fibrinogen and or the thrombin may be activated by a suitable, physiologically-acceptable liquid, optionally containing any necessary co-factors and/or enzymes, prior to or during application of the hemostatic sandwich bandage to the wounded tissue.

In addition, one or more supplements may also be contained in one or more layers of the inventive hemostatic sandwich bandage, such as growth factors, drugs, polyclonal and monoclonal antibodies and other compounds. Illustrative examples of such drugs include, but are not limited to: antibiotics, such as tetracycline and ciprofloxacin, amoxicillin, and metronidazole: anticoagulants, such as activated protein C, heparin, prostacyclin (PGI₂), prostaglandins, leukotrienes, antithrombin III, ADPase, and plasminogen activator; steroids. such as dexamethasone, inhibitors of prostacyclin, prostaglandins, leukotrienes and/or kinins to inhibit inflammation; cardiovascular drugs, such as calcium channel blockers, vasodilators and vasoconstrictors; chemoattractants; local anesthetics such as bupivacaine; and antiproliferative/antitumor drugs such as 5-fluorouracil (5-FU), taxol and/or taxotere; antivirals, such as gangcyclovir, zidovudine, amantidine, vidarabine, ribaravin, trifluridine. acyclovir, dideoxyuridine and antibodies to viral components or gene products; cytokines, such as α- or β- or γ-Interferon, α- or β-tumor necrosis factor, and interleukins; colony stimulating factors; erythropoietin; antifungals, such as diflucan, ketaconizole and nystatin; antiparasitic agents, such as pentamidine; anti-inflammatory agents, such as α-1-anti-trypsin and α-1-antichymotrypsin; anesthetics, such as bupivacaine; analgesics; antiseptics; and hormones. Other illustrative supplements include, but are not limited to: vitamins and other nutritional supplements; glycoproteins; fibronectin: peptides and proteins; carbohydrates (both simple and/or complex); proteoglycans; antiangiogenins; antigens; lipids or liposomes; oligonucleotides (sense and/or antisense DNA and/or RNA); and gene therapy reagents.

The following examples are illustrative only and are not intended to limit the scope of the invention as defined by the appended claims. It will be apparent to those skilled in the art that various modifications and variations can be made in the methods of the present invention without departing from the spirit and scope of the invention. Thus, it is intended that the present invention cover the modifications and variations of this invention provided they come within the scope of the appended claims and their equivalents.

All patents and publications referred to herein are expressly incorporated by reference.

### V. EXAMPLES

### A. EXAMPLE I

Fibrinogen and thrombin vials were removed from refrigerator and allowed to warm to room temperature for 2 hours. To each vial of thrombin 2mL of 40mM CaCl₂ was added, this yielded a final concentration of 500U/mL of thrombin. Dispo molds (Baxter) size 3.0 X 2.4 cm were placed on freezing tray on top of dry ice.

To each of 12 molds, 1.75 mL of H₂O was added and allowed to freeze for 1 hour at -80°C. Once frozen 140µL of thrombin (70 units) was pipetted on top of the H₂O and allowed to freeze for an additional 1 hour at -80°C. Fibrinogen was solubilized with water (8mL) to a concentration of 50mg/mL. To the molds 1.1 mL of fibrinogen was added (55mg) and allowed to freeze for an additional 1 hour at -80°C, followed by the addition of 1mL of H₂O. The bandages were allowed to freeze for 1 hour at -80°C. Then 0.25ml of thrombin (125 units) was added and allowed to freeze for one hour. An additional 1.1 mL of fibrinogen (55mg) was pipetted on top and allowed to freeze for 1 hour.

Once all materials were added and frozen, VICRYL was placed on top and pressed into placed, by gentle finger pressure. To cover the VICRYL 500µL of 30% sucrose was added and allowed to freeze. The bandages were placed at -80°C for 2 hours, then placed into the freeze dryer. Upon completion. vacuum was released and the bandages were removed and examined.
RESULTS: The bandages when removed from the freeze dryer were uniform and consistent, the VICRYL was attached and all layers were intact.

### B. EXAMPLE II

The fibrinogen and thrombin vials were removed from the refrigerator and allowed to warm to room temperature for 2 hours. Vicryl™ was applied and pressed into Dispo plastic molds (2.4cm x 2.4cm). The molds were incubated at -80°C for 1 hour.

Fibrinogen was solubilized with 21 mL of 2% sucrose or water to a concentration of 19.2mg/mL. In addition, fibrinogen was solubilized to a concentration of 1mg/mL with either 2% sucrose or water. The molds received 1.23mL of fibrinogen and then were frozen at -80°C for 1 hour.

Thrombin was solubilized with 0.5mL of 40mM CaCl₂ to a concentration of 2000U/mL. The bandages were removed from the freezer and placed on dry ice. Thrombin was sprayed at 37.5U/cm². The bandages were returned to -80°C for an additional 1 hour.

The bandages were placed on dry ice and received a second layer of fibrinogen (identical to the first layer). Half of the bandages were returned to -80°C for 2 hours and the other half of the bandages were placed at -20°C for 2 hours. Then, the bandages were placed in the freeze-dryer.

The bandages were subjectively assessed for resorbable material adherence and bandage appearance. Table 3 summarizes the experimental design.

**Table 3.**

| **Various bandage configurations and formulations** | | | | | |
|---|---|---|---|---|---|
| **Bottom Layer** | **Second Layer** | **Third Layer** | **Fourth Layer** | **Freeze temp prior to** placement **into freeze-dryer** | **Bandage group** |
| Fibrinogen 1mg/cm² in 2% sucrose | VICRYL™ | Thrombin in 40mM CaCl₂ | Fibrinogen 8mg/cm² in 2% sucrose | -20°C or -80°C | 1 |
| Fibrinogen 1mg/cm² in water | VICRYL™ | Thrombin in 40mM CaCl₂ | Fibrinogen 8mg/cm² in 2% sucrose | -20°C or -80°C | 2 |
| Fibrinogen 8mg/cm² in 2% sucrose | VICRYL™ | Thrombin in 40mM CaCl₂ | Fibrinogen 8mg/cm² in 2% sucrose | -20°C or -80°C | 3 |
| Fibrinogen 8mg/cm² in water | VICRYL™ | Thrombin in 40mM CaCl₂ | Fibrinogen 8mg/cm² in 2% sucrose | -20°C or -80°C | 4 |
| VICRYL™ | Fibrinogen 1mg/cm² in 2% sucrose | Thrombin in 40mM CaCl₂ | Fibrinogen 8mg/cm² in 2% sucrose | -20°C or -80°C | 5 |
| VICRYL™ | Fibrinogen 1mg/cm² in water | Thrombin in 40mM CaCl₂ | Fibrinogen 8mg/cm² in 2% sucrose | -20°C or -80°C | 6 |
| VICRYL™ | Fibrinogen 8mg/cm² in 2% sucrose | Thrombin in 40mM CaCl₂ | Fibrinogen 8mg/cm² in 2% sucrose | -20°C or -80°C | 7 |
| VICRYL™ | Fibrinogen 8mg/cm² in water | Thrombin in 40mM CaCl₂ | Fibrinogen 8mg/cm² in 2% sucrose | -20°C or -80°C | 8 |

RESULTS: The results from table 3 showed that the best bandage was. This was true for bandages frozen at both -20°C or -80°C.

### C. EXAMPLE III

This bandage configuration had Vicryl™ and thrombin "layered" in between two layers of fibrinogen. The first layer of fibrinogen was solubilized in either 2% sucrose or water. The bandages were frozen at -20°C or -80°C prior to placement into the freeze-dryer. The experimental design is outlined in Table 4. The bandages were subjectively observed for physical appearance after freeze-drying and handling characteristics after hydration.

**Table 4.**

| **"Layered" bandage configuration** | | | | | | |
|---|---|---|---|---|---|---|
| **Bottom Layer** | **Second Layer** | **Third Layer** | **Fourth Layer** | **Fifth Layer** | **Freeze temp prior to placement into freeze-dryer** | **Group** |
| 2% Sucrose | Fibrinogen 1mg/cm² in 2% sucrose | VICRYL™ | Thrombin in 40mM CaCl₂ | Fibrinogen 8mg/cm² in 2% sucrose | - 20°C or - 80°C | 1 |
| 2% Sucrose | Fibrinogen 1mg/cm² in water | VICRYL™ | Thrombin in 40mM CaCl₂ | Fibrinogen 8mg/cm² in 2% sucrose | - 20°C or - 80°C | 2 |
| 2% Sucrose | Fibrinogen 8mg/cm² in 2% sucrose | VICRYL ™ | Thrombin in 40mM CaCl₂ | Fibrinogen 8mg/cm² in 2% sucrose | - 20°C or - 80°C | 3 |
| 2% Sucrose | Fibrinogen 8mg/cm² in water | VICRYL ™ | Thrombin in 40mM CaCl₂ | Fibrinogen 8mg/cm² in 2% sucrose | - 20°C or - 80°C | 4 |

RESULTS: Two structures for layered bandage production; group 7 at -20°C and -80°C from table 3 and group 1 at -20°C and -80°C from table 4, were exhibited the best characteristics in terms of no separation of layers and attachment of resorbable material.

### D. EXAMPLE IV

Fibrinogen and thrombin were removed from the refrigerator and allowed to warm to room 25°C for 2 hours. For groups 1 + 2 each vial of fibrinogen received 15.3mL of 2% sucrose, final concentration of 26.2mg/mL; bandages for group 3 had 21mL of 2% sucrose to give a final concentration of 19.2mg/mL. The final amount of fibrinogen was 8mg/cm² for all three groups.

Dispo molds (3.0 X 2.4 cm) were placed on a freezing tray on top of dry ice. 1mL of 2% sucrose was added and allowed to freeze at -80°C for 1 hour, once frozen VICRYL was placed on top and pressed into place. Groups 1 + 2 received 2.2mL of fibrinogen, whereas group 3 received 1.5mL of fibrinogen..

All bandages were allowed to freeze at -80°C for 2 hours. Group 1 was kept at -80°C until placed into the freeze dryer. Thrombin was solubilized with 0.5mL of 40mM CaCl₂ to a concentration of 2000U/mL. Groups 2 & 3 were sprayed with thrombin, so that each bandage received 144U/bandage while on dry ice. Once thrombin was applied the bandages were placed at -80°C for 1 hour. Group 3 was removed from the freezer and placed on dry ice, and an additional 1.5mL of fibrinogen at 19.2mg/mL was added.

When finished all bandages were returned to -80°C for another 2 hours. The bandages were then place into the freeze dryer, after 96 hours a sample of each group of bandages were removed and moisture content determined. Secondary drying was initiated for 24 hours. When the bandages were removed, moisture content was measured again.
RESULTS: As can be seen in table 5 the moisture content of the bandages decreases with secondary drying. All bandages looked identical and had the same texture.

**Table 5**

| **Bandage Group** | **% Moisture** | |
|---|---|---|
| | Pre-Secondary Drying | Post-Secondary Drying |
| Fibrinogen alone (1) | 4.2 | 3.8 |
| Fibrinogen/Thrombin (2) | 4.6 | 3.3 |
| Fibrinogen/Thrombin/Fibrinogen (3) | 4.3 | 3.9 |

### E. EXAMPLE V

Fibrinogen and thrombin were removed from the refrigerator and allowed to warm to room 25°C for 2 hours. Dispo molds of size 3.0 X 2.4 cm were sprayed with 300µL of 2% sucrose on dry ice, VICRYL was applied and pressed into place and the molds placed at -80°C for 1 hour. Fibrinogen was solubilized with 11 mL of 2% sucrose, to a concentration of 36.4mg/mL. Each bandage had a final fibrinogen amount of 15mg/cm².

The molds received 1.5mL of fibrinogen and then were frozen at -80°C for 1 hour. Thrombin was solubilized with 0.5mL of 40mM CaCl₂ to a concentration of 2000U/mL. All bandages were sprayed with thrombin, while on dry ice and received 37.5U/cm².

The bandages were returned to -80°C for an additional 1 hour. The bandages were placed on dry ice and received a second layer of fibrinogen, identical to the first layer. The bandages were again returned to -80°C for 2 hours, until placed into the freeze dryer.
RESULTS: The bandages were removed from the freeze dryer and a sample of the 3.0 X 2.4 cm bandages were analyzed for moisture content. These bandages had a moisture content of 2.49%. The bandages were complete and had no separation of layers, and the VICRYL was well attached.

### F. EXAMPLE VI

Forty-four square petri dishes with a size of 10.1 X 10.1 cm, with a surface area of 103cm² were placed on shelf trays and received 20mL of 2% sucrose, which is equal to 194µL/cm². Once the molds were filled they were placed at -80°C for 2 hours until frozen, and then VICRYL applied.

Fibrinogen and thrombin were removed from the refrigerator and allowed to warm to 25°C for 2 hours. Each vial of fibrinogen was solubilized with 10mL of 2% sucrose. The concentration of fibrinogen when reconstituted was 16mg/mL. The molds received 25mL of fibrinogen and where returned to -80°C for 2 hours until frozen. Thrombin was solubilized with 0.5mL of 40mM CaCl₂ to a concentration of 2000U/mL, and sprayed on top of the fibrinogen and returned to -80°C for 1 hour. An additional 25mL of fibrinogen was added and allowed to freeze at -80°C for 2 hours.

The bandages were then freeze dried, upon completion the bandages were packaged and sent for *in vivo* testing. described *supra*.

### G. EXAMPLE VII

Fibrinogen and thrombin were removed from the refrigerator and allowed to warm to 25°C for 2 hours. Dispo plastic molds (3.0cm x 2.4cm) were placed on dry ice and sprayed with 300µL of 2% sucrose. Vicryl™ or calcium alginate was applied and pressed into place. The molds were incubated at -80°C for 1 hour.

The calcium alginate was used in two ways. In the first method, the calcium alginate was cut to the same size section as the Vicryl™ sections. In the second method, the calcium alginate was also cut to the same size section as the Vicryl™ sections. but then it was shredded into fine pieces of material (Table 6).

Fibrinogen was solubilized with 11mL of 2% sucrose to a concentration of 36.4mg/mL. Each bandage had a final fibrinogen amount of 15mg/cm². The molds received 1.55mL of fibrinogen and then were frozen at -80°C for 1 hour. Thrombin was solubilized with 0.5mL of 40mM CaCl₂ to a concentration of 2000U/mL. The bandages were placed on dry ice and sprayed with thrombin to yield 37.5U/cm².

The bandages were returned to -80°C for 2 hours until they were placed into the freeze-dryer. After the bandages were freeze-dried, they were tested in the porcine arteriotomy bandage performance test.

**Table 6.**

| **Bandage resorbable material combinations using Vicryl™ and Calcium alginate** | | |
|---|---|---|
| **Bandage** | **Vicryl** | **Calcium Alginate** |
| 1 | No | Whole |
| 2 | No | Shredded |
| 3 | Yes | Shredded |
| 4 | Yes | Whole |

### Porcine Arteriotomy Bandage Performance Test

Obtain frozen porcine aorta and thaw. Aortas can be thawed overnight at 4°C, or individually wrapped in the water bath at 37°C. Dissect excess connective tissue from approximately first 11 cm of the aorta. Usually, the first 5-5.5 cm are free from collateral vessels. The next 5-5.5 cm should not have more than 1-2 collaterals. These can be easily sealed or patched with cyanoacrylate glue.

Cut the aorta into two 5.5 cm pieces. Invert aorta exposing the interior using a hemostat or blunt forceps. Wash both the interior and exterior of the vessel with 1-5 mL of PBS at 37°C. Stretch an O-ring over a 20cc syringe with an approximately 0.6 cm (0.25 in) hole drilled into one side. Using fingers or hemostats pull the vessel onto the syringe. Fit another O-ring of the same size onto the bottom.

Using curved hemostats, carefully secure both O-rings over the top of the vessel. The distance between both O-rings should be 3.5 cm. The artery should be snug fitting and held securely in place. Position the secured vessel such that the hole in the syringe lies in the middle of the distance between the O-rings.

Fill the syringe with PBS at 37°C and place the screw through the outside of the syringe and into the plunger, so that the plunger is held in a stationary position. Wash the artery on the syringe with 1-2ml of PBS at 37°C. Using a 16-gauge needle, make a hole in the center (approximately 1.75 cm from either O-ring) over the syringe hole. The 16-gauge needle should be replaced after every 12 uses.

Open the sealed bag containing the bandage and immediately place the bandage over the incision (approximately 0.5 cm from each O-ring, so as not to touch either O-ring). All bandages should be individually packaged prior to use.

Using a P-1000 Pipetman, wet the bandage with PBS at 37°C. For 15 mg/cm² bandages use 800µL, and for 8 mg/cm² bandages use 500µL. Immediately place the syringe shield on top of the bandage, so as not to touch either O-ring. Press lightly to secure.

Place the syringe into the incubator at 37°C using the holding box in order to keep the syringe and all its components stationary. Cover with the plastic cover, placing a 200g weight securely over top. Assure even distribution of weight. Allow it to incubate for 5 minutes at 37°C.

Remove the syringe from the incubator. Carefully remove the shield covering the bandage. Attach the syringe to the tubing connected to a peristaltic pump. The tubing should be arranged so that it runs through the pump and is connected to a Y-junction on the opposite side. The Y-junction creates two outlets. allowing the PBS to be pumped into the syringe at one site as back pressure is being generated in the other. This back pressure is directly measured using an in-line pressure transducer-analyzer, and recorded using DMSI-200/1 software.

Pump the PBS at 37°C into the syringe. and immediately start monitoring the pressure generated. Initiate a 30-second slow ramp (setting 4 at 1 x speed for pump in E229, setting 7.5 at 1x speed for pump in E132), such that the initial flow rate is approximately 0.3 mL/min.

After the first 30 seconds, the flow rate is escalated to approximately 3 mL/min (10x speed, both settings). This should be done until a pressure of 200 mm Hg is obtained. Once 200 mm Hg is achieved, start the timer for 2 minutes.

Stop the pump once 200 mmHg is obtained. Monitor the pressure generated. If pressure starts to drop. turn pump back on until adequate pressure is obtained. This may be done as often as necessary throughout the two-minute interval (under normal conditions, the pressure should be maintained between 200 and 215 mm Hg.). In addition, note any leakage and its location. If a leak occurs. note the maximum time and burst pressure at the moment of leakage. Judge bandage performance based on the following pass / fail criteria.

A bandage is considered passing if it maintains a fairly consistent pressure of 200 mm Hg for two minutes with absolutely no leakage. A bandage is also considered passing if it maintains a fairly consistent pressure of 200 mm Hg for two minutes with only minimal leakage (e.g., slow seeping or a leak that has resealed itself).

A bandage is considered failing if it cannot maintain adequate pressure due to severe leakage. This includes leakage caused by poor adhesion, as well as leakage due to manufacturing flaws.
RESULTS: The results of the experiment showed that bandages can be prepared using calcium alginate as a resorbable material or used as an additional component to the fibrinogen. There was no difference in the appearance of the bandages when removed from the freeze dryer. In addition, all groups of bandages when tested in the porcine arteriotomy assay held 200mm Hg for 3 minutes.

### H. EXAMPLE VIII

Fibrinogen and thrombin were removed from the refrigerator and allowed to warm to 25°C for 2 hours. Dispo plastic molds (2.4cm x 2.4cm) were placed on dry ice and sprayed with 300µl of 2% sucrose. Some of the molds had Vicryl™ applied and pressed into place and other molds had Gelfoam® applied and pressed into place as a resorbable material.

Fibrinogen was solubilized with 11mL of 2% sucrose to a concentration of 36.4mg/mL for the 15mg/cm² bandages. Collagen Type I was solubilized with 95% ethanol to a concentration of 36.4mg/mL.

In addition to the 15mg/cm² bandages, 8mg/cm² bandages were also prepared. The molds received 1.23mL of fibrinogen or collagen and then were frozen at -80°C for 1 hour. Thrombin was solubilized with 0.5mL of 40mM CaCl₂ to a concentration of 2000U/mL. The bandages were placed on dry ice and sprayed with thrombin.

The bandages were returned to -80°C for an additional hour. Then the bandages were placed on dry ice and received a second layer of fibrinogen (identical to the first layer). The bandages were returned to -80°C for 2 hours until they were placed into the freeze-dryer.

Table 7 summarizes the experimental design. After freeze-drying, the bandages were subjectively assessed for their physical appearance.

**Table 7.**

| **Formulation and configuration of bandages using different resorbable materials and different protein components** | | | | |
|---|---|---|---|---|
| **1st Layer** | **2nd Layer** | **3rd Layer** | **4th Layer** | **5th Layer** |
| 2% sucrose | Vicryl | Collagen I 15mg/cm² | Thrombin | NA |
| 2% sucrose | Vicryl | Collagen I 8mg/cm² | Thrombin | NA |
| 2% sucrose | NA | Collagen I 15mg/cm² | Thrombin | NA |
| 2% sucrose | NA | Collagen I 8mg/cm² | Thrombin | NA |
| 2% sucrose | Vicryl | Fibrinogen 15mg/cm² | Thrombin | Fibrinogen 15mg/cm² |
| 2% sucrose | Vicryl | Fibrinogen 8mg/cm² | Thrombin | Fibrinogen 8mg/cm² |
| 2% sucrose | Vicryl | Fibrinogen 7.5mg/cm² + Collagen 7 5mg/cm² | Thrombin | Fibrinogen 7.5mg/cm² + Collagen 7.5mg/cm² |
| 2% sucrose | Vicryl | Fibrinogen 4mg/cm² + Collagen 4mg/cm² | Thrombin | Fibrinogen 4mg/cm² + Collagen 4mg/cm² |
| 2% sucrose | Gelfoam | Thrombin | NA | NA |
| 2% sucrose | Gelfoam | Fibrinogen 15mg/cm² | | Fibrinogen 15mg/cm² |
| 2% sucrose | Gelfoam | Fibrinogen 8mg/cm² | | Fibrinogen 8mg/cm² |

RESULTS: After freeze drying the bandages were evaluated for their physical appearance. All combinations manufactured produced satisfactory bandages. In that they were all intact and had no separation of layers.

### I. EXAMPLE IX

Fibrinogen and thrombin were removed from the refrigerator and allowed to warm to 25°C for 2 hours. Dispo plastic molds (3.0cm x 2.4cm) were placed on dry ice and sprayed with 300µL of 2% sucrose. Vicryl™ was applied and pressed into place. The molds were incubated at -80°C for 1 hour.

Fibrinogen was solubilized with 11mL of 2% sucrose to a concentration of 36.4mg/mL. Each bandage had a final fibrinogen amount of 15mg/cm². Each group of bandages received their pre-determined designated amount of fibrinogen (as listed in Table 8). The bandages were then frozen at -80°C for 1 hour.

**Table 8.**

| **Bandage configurations with various percentages of fibrinogen** | | | | |
|---|---|---|---|---|
| **1st Layer: % Fibrinogen** | **Volume (ml) Fibrinogen** | **% Thrombin** | **2nd Layer: % Fibrinogen** | **Volume (ml) Fibrinogen** |
| 100 | 3.1 | 100 | 0 | 0 |
| 0 | 0 | 100 | 100 | 3.1 |
| 75 | 2.325 | 100 | 25 | 0.775 |
| 25 | 0.775 | 100 | 75 | 2.325 |
| 50 | 1.55 | 100 | 50 | 1.55 |

Thrombin was solubilized with 0.5mL of 40mM CaCl₂ to a concentration of 2000U/mL. The bandages were placed on dry ice and sprayed with thrombin at 37.5U/cm².

The bandages were returned to -80°C for an additional hour. The bandages were placed on dry ice and received a second layer of fibrinogen (as listed in Table 8). The bandages were returned to -80°C for 2 hours until they were placed into the freeze-dryer.

After freeze-drying, the bandages were tested in the porcine arteriotomy test. Bandages were tested on day 0 and two months post manufacturing after room temperature storage in foil bags.

**Table 9.**

| **Porcine arteriotomy test results with various percentages of fibrinogen bandages** | | | | |
|---|---|---|---|---|
| **% Fibrinogen (Bottom Layer)** | **% Thrombin (Middle Layer)** | **% Fibrinogen (Top Layer)** | **% Bandages that Passed Porcine Arteriotomy Test:** | |
| | | | **Time 0** | **Time 2 months** |
| 100 | 100 | 0 | 0 | 0 |
| 0 | 100 | 100 | 16.6 | 33.375 |
| 75 | 100 | 25 | 50 | 66.67 |
| 25 | 100 | 75 | 50 | 66.67 |
| 50 | 100 | 50 | 100 | 100 |

RESULTS: These results clearly show that the bandage produced with fibrinogen at 50% for the first layer and 50% for the second layer (fibrinogen 50/50 bandages) performs far superior to all other tested combinations of the bandages in this porcine arteriotomy model. Both at time 0 and 2 months, 100% of the fibrinogen 50/50 bandages passed the porcine arteriotomy test.

### J. EXAMPLE X

Fibrinogen and thrombin were removed from the refrigerator and allowed to warm to 25°C for 2 hours. Dispo plastic molds (2.4cm x 2.4cm) were placed on dry ice and sprayed with 300µL of 2% sucrose. Vicryl™ was applied and pressed into place and the molds were placed at -80°C for 1 hour.

Fibrinogen was solubilized with 11mL of 2% sucrose to a concentration of 36.4mg/mL. Each bandage had a final fibrinogen amount of 15mg/cm². The molds received 1.23mL of fibrinogen and then were frozen at -80°C for 1 hour.

Thrombin was solubilized with 40mM CaCl₂, 13mM CaCl₂, or 4mM CaCl₂. The bandages were placed on dry ice and sprayed with thrombin at either 37.5U/cm², 12.5U/cm², 4.2U/cm², or 1.4U/cm². The bandages were returned to -80°C for an additional hour.

The bandages were placed on dry ice and received a second layer of fibrinogen (identical to the first layer). The bandages were returned to -80°C for 2 hours until they were placed into the freeze-dryer. After the bandages were freeze-dried, they were evaluated for physical appearance and tested in the porcine arteriotomy test.
RESULTS: In this experiment, the bandages were produced using 40mM, 13mM, or 4mM CaCl₂ to solubilize the thrombin and the bandages received thrombin at 37.5U/cm², 12.5U/cm², 4.2U/cm², or 1.4U/cm². Table 10 shows the experimental design and the results.

**Table 10.**

| **Bandage (with variations in thrombin amount and CaCl**_{**2**} **concentration) performance in the pig arteriotomy test** | | | |
|---|---|---|---|
| **Thrombin Units/cm**^{**2**} | **CaCl**_{**2**} **mM** | **Results** | |
| | | **% Bandages Separated Following Freeze Drying** | **% Bandages Pass Pig Arteriotomy** |
| 1.4 | 4.4 | 100 | 0 |
| 4.2 | 4.4 | 100 | 0 |
| 12.5 | 4.4 | 100 | 0 |
| 37.5 | 4.4 | 0 | 0 |
| 1.4 | 13 | 87.5 | 50 |
| 4.2 | 13 | 25 | 0 |
| 12.5 | 13 | 12.5 | 75 |
| 37.5 | 13 | 0 | 100 |
| 1.4 | 40 | 87.5 | 0 |
| 4.2 | 40 | 12.5 | 83 |
| 12.5 | 40 | 12.5 | 100 |
| 37.5 | 40 | 0 | 100 |

The results from Table 10 show that the best combination for the bandage is 37.5U/cm² of thrombin solubilized in either 13mM or 40mM CaCl₂. This combination is superior to all other combinations tested in both physical appearance and ability to pass the porcine arteriotomy test. These two combinations of the bandage exhibited no separation of the component layers after freeze-drying and 100% of the bandages passed the porcine arteriotomy test.

It should be noted that there were no adjustments made in the freeze-drying cycle when these bandages were manufactured so it is possible the cycle could be modified in such a way to consistently prepare bandages with lower thrombin and/or CaCl₂ amounts. However, a decrease in the amount of thrombin would prolong the amount of time needed for fibrin formation and may jeopardize the quality of the bandage, especially in case of trauma where the temperature of the body may be lower than normal thereby decreasing the bodies own clotting ability.

### K. EXAMPLE XI

Fibrinogen and thrombin were removed from the refrigerator and allowed to warm to 25°C for 2 hours. Dispo plastic molds (2.4cm x 2.4cm) were placed on dry ice and sprayed with 300µl of 2% sucrose. Vicryl™ was applied and pressed into place. The molds were placed at -80°C for 1 hour.

Fibrinogen was solubilized with 11mL of 2% sucrose to a concentration of 36.4mg/mL for 15mg/cm². 21mL of 2% sucrose to a concentration of 18.7mg/mL for 8mg/cm², 42.8mL of 2% sucrose to a concentration of 9.35mg/mL for 4mg/cm², and 85.6mL of 2% sucrose to a concentration of 4.68mg/mL for 2mg/cm². The molds received 1.23mL of fibrinogen and then were frozen at -80°C for 1 hour.

Thrombin was solubilized with 0.5mL of 40mM CaCl₂ to a concentration of 2000U/mL. The bandages were placed on dry ice and sprayed with thrombin.

The bandages were returned to -80°C for an additional hour. The bandages were placed on dry ice and received a second layer of fibrinogen (identical to the first layer).

The bandages were returned to -80°C for 2 hours until they were placed into the freeze-dryer. After the bandages were freeze-dried, they were observed for their physical appearance and tested in the porcine arteriotomy test.

**Table 11.**

| **% Bandages That Maintained 200mm Hg for 3 minutes in Porcine Arteriotomy Assay** | |
|---|---|
| **Fibrinogen Concentration mg/cm**^{**2**} | **% Bandages to Maintain 200mm Hg** |
| 15 | 100 |
| 8 | 50 |
| 4 | 37.5 |
| 2 | 18.75 |

### L. EXAMPLE XII

Fibrinogen and thrombin were removed from the refrigerator and allowed to warm to 25°C for 2 hours. Dispo plastic molds (2.4cm x 2.4cm) were placed on dry ice and sprayed with 300µL of 2% sucrose.

Fibrinogen was solubilized with 11mL of 2% sucrose to a concentration of 36.4 mg/mL. Each bandage had a final fibrinogen amount of 15mg/cm². The molds received 1.23mL of fibrinogen and then were frozen at -80°C for 1 hour.

Thrombin was solubilized with 40mM CaCl₂, the bandages were placed on dry ice and sprayed with thrombin at 37.5U/cm². The bandages were returned to -80°C for an additional hour.

The bandages were placed on dry ice and received a second layer of fibrinogen (identical to the first layer). VICRYL™ was applied and pressed into place and the molds were sprayed with 300µL of 2% sucrose. The bandages were returned to -80°C for 2 hours until they were placed into the freeze-dryer.
RESULTS: The bandages were evaluated for physical appearance when removed from the freeze dryer. Although the bandages layers were intact the VICRYL was not attached to the bandage.

### M. EXAMPLE XIII

Fibrinogen was removed from the refrigerator and allowed to warm to 25°C for 2 hours. Dispo plastic molds (2.4cm x 2.4cm) were placed on dry ice and sprayed with 300µL of 2% sucrose. Calcium alginate was applied and pressed into place.

Fibrinogen was solubilized with 11mL of 2% sucrose to a concentration of 36.4 mg/mL. Each bandage had a final fibrinogen amount of 15mg/cm². The molds received 2.4 mL of fibrinogen and then were frozen at -80°C for 2 hours, then placed into the freeze dryer.
RESULTS: The bandages when removed from the freeze dryer were intact.

### N. EXAMPLE XIV

Fibrinogen and thrombin were removed from the refrigerator and allowed to warm to 25°C for 2 hours. Dispo plastic molds (2.4cm x 2.4cm) were placed on dry ice and sprayed with 300µl of 2% sucrose. Vicryl™ was applied and pressed into place. Fibrinogen was solubilized with 11mL of 2% sucrose to a concentration of 36.4 mg/mL; in addition each vial received 0.2 grams of CHAPS. Each bandage had a final fibrinogen amount of 15mg/cm². The molds received 1.23mL of fibrinogen/CHAPS and then were frozen at -80°C for 1 hour.

Thrombin was solubilized with 40mM CaCl₂; the bandages were placed on dry ice and sprayed with thrombin at 37.5U/cm^{2.} The bandages were returned to -80°C for an additional hour.

The bandages were placed on dry ice and received a second layer of fibrinogen/ CHAPS (identical to the first layer). The bandages were returned to -80°C for 2 hours until they were placed into the freeze-dryer.
RESULTS: The bandages when removed from the freeze dryer were intact and uniform in shape.

### O. EXAMPLE XV

The mean burst pressure of bandages incorporating different backing material and a fibrinogen concentration of 15 mg/cm² was determined.

| **BACKING MATERIAL** | **FIBRINOGEN CONCENTRATION** | **MEAN BURST PRESSURE (mm Hg)** | **ADHERENCE TO BACKING MATERIAL** |
|---|---|---|---|
| DEXON #2 2000 micron | 8mg/cm² | 15 | NO |
| DEXON #4 230 micron | 8mg/cm² | 250* | YES |
| DEXON #8 100 micron | 8mg/cm² | 181 | NO |
| VKML KNITTED MESH | 8mg/cm² | 250* | YES |
| VWML WOVEN MESH | 8mg/cm² | 210 | NO |

| | | | |
|---|---|---|---|
| * All of the bandages maintained mean burst pressure for the maximum test period of 3 minutes. | | | |

RESULTS: Maximum mean burst pressure and time was achieved with bandages at a concentration of 8 mg/cm². The mean burst pressure for VICRYL and Dexon meshes alone is zero.

### P. EXAMPLE XVI: SUMMARY OF IN VIVO DATA

### Layered (fibrinogen 50/50) bandage application methods

Layered fibrinogen/thrombin/fibrinogen bandages at 15 mg/cm² fibrinogen were tested in the rat arterial hemorrhage model using two distinct application methods for the bandage: 1) the 500 g weight on a molded platform was used to apply pressure for fifteen minutes and with the variation that an additional one minute of pressure was applied if bleeding was noted or 2) finger pressure was applied for 7 minutes, and in the latter part of the study with the option to apply a second bandage with finger pressure for an additional 3 minutes if bleeding was noted after the first bandage. In all cases by either method, the bandage was applied to a dry field and the blood flow from the artery was used to soak the bandage.

### Rat Hemorrhage Model

**Animals:** Model: Rat: Vendor: Harlan Sprague Dawley; Strain: Sprague Dawley; Color: White; Age:120 days to 180 days: Sex: Male; Weight: 400g to 500g.

### Anesthesia:

### 1) 1.1 g/Kg of 55% w/v Urethane (methyl carbamate) given IP

a) Urethane supplied by: Sigma (lot 55H0368)
b) Urethane prepared fresh daily by weighing out 2.0g urethane into a sterile glass rubber-top tube (Becton Dickson. Vacutainer, 5 ml Draw Lot # 6A715 exp. Dec 97). An equal weight / volume (2.0 cc) sterile water for injection (Abbot Laboratories, 10ml Sterile Water for Injection, U.S.P., Lot 11-221-DK. Exp Dec 1, 97) is added to produce a final solution volume of 3.6 mL (producing a solution of 55.56% urethane). This preparation procedure is performed in a biological safety cabinet (Forma Scientific, model 1168, serial no. 14088-43).
c)The urethane injection is drawn into, and administered with, a 1cc syringe(Becton Dickson, tuberculin 1cc syringe, sterile Lot no. 3E345) with a 23 gauge, 3/4 inch needle (Becton Dickson, PrecisionGlide needle, sterile Lot no. 4B047). The rat is weighed, manually restrained, and the urethane is injected intraperitoneally (IP) into the left caudal quadrant of the ventral abdomen. The volume injected ranges from 0.80 to 1.00 mL for rats weighing 400 to 500 g to deliver a dose of 1.1 g/Kg.
d) Time clock started immediately after urethane injection recorded as T=0

### 2) 0.06 mg/Kg atropine and 0.15 mg/Kg buprenorphine given together 1M.

a) Atropine sulfate supplied by: Phoenix Pharmaceutical, Inc. (Lot# 5110547, Exp.11/96)
b) Buprenorphine HCl (Buprenex) supplied by: Reckitt & Colman Pharmaceuticals (CN#3555, Exp. 1, Feb. 99)
c) Atropine is drawn first into a 1cc syringe (ibid) with a 26 gauge, 1/2 inch needle (Becton Dickson. PrecisionGlide needle, sterile Lot no. 4B046), then the buprenorphine is drawn into the same syringe. The rat is given an intramuscular injection in the caudal thigh with the same syringe and needle when it has lost its righting reflex (2 - 3 minutes after the urethane injection). The volume injected is 0.04 to 0.05 mL of atropine. and 0.20 to 0.25 mL of buprenorphine, making a total volume of 0.24 to 0.30 mL for 400 - 500g rats.
d) Time of injection recorded to nearest minute

### Surgery:

### 1) Shave animal:

a) Right axillary region for placement of thermoprobe
b) Ventral Neck for right carotid artery cannulation
c) Ventral abdominal midline for laparotomy

### 2) Thermoprobe placement:

a) Stab incision right axilla with #15 or # 17 scalpel blade
b) Subcutaneous tissue undermined caudal to incision with Hartman mosquito forceps.
c) Thennoprobe (Traceable™, Certification of Calibration for DigitalThermometer, Model Number 15-078-39. Serial Number 566680 ) placed subcutaneously and temperature recorded
d) Time of thermoprobe placement recorded to nearest minute
e) Body temperature is maintained between 33 and 39 C by use of electric heat support

### 3) Carotid arterial cannulation/pressure transducer placement (under operating microscope):

a) Ventral neck incised with from manubrium to mandibular symphysis -- time recorded
b) Cervical musculature bluntly dissected along fascial planes, exposing right carotid artery
c) Carotid artery bluntly dissected from surrounding tissue and distracted ventrally
d) Distal end of carotid exposure ligated with 4-0 silk, proximal end elevated with a length of 4-0 silk, and a third length of 4-0 silk placed under the artery midway between.
e) Beveled arteriotomy made with microvascular scissors and the transducer/catheter (Millar Mikro-Tip Catheter Pressure Transducer System. Size 2F, Model SPR-407) inserted into the artery 2-3 cm proximally, then ligated using the third length of silk with a quick release knot.
f) Instruments and elevating suture removed from the field, neck incision partially closed with a staple,andthe rat situated in preparation for the laparotomy
g) Computerized data collection is begun -- time recorded.*
h) At least five sets of data recorded at one minute intervals (mean arterial pressure, systolic and diastolic pressures, and heart rate readings), averaged over each minute.

### 4) Laparotomy (under operating microscope):

a) Ventral midline incision from xiphoid to pubis through skin and linea alba.
b) Abdominal incision retracted with Weitlaner Retractor (3x4 8"sharp) , small intestine exteriorized to right side of animal and covered with warm saline moistened gauze. colonic mesentery bluntly dissected and colon displaced to the right.
c) Retroperitoneum bluntly dissected, exposing lumbar region of aorta/vena cava distal to the renal arteries. Adipose tissue cleaned away from vessel complex and surrounding muscle bed. creating a window approximately 2 - 3 cm diameter.
d) Diameter of aorta measured with micrometer calipers and documented.
e) Aorta clamped with Jacobson micro mosquito hemostatic forceps at cranial edge of window just distal to the renal arteries and clamped with Dieffenbach Micro Clamp at the caudal edge of window.
f) A 4 mm longitudinal incision is made using a microvascular scalpel and arteriotomy scissors on the ventral aspect of the aorta at the level of, or just distal to, the large lumbar veins. Incision measured with micrometer calipers and an ocular rule installed in the microscope eyepiece is utilized to guide the incision-making process.
g) Distal aortic clamp removed, proximal aortic clamp briefly released allowing approx. 0.5 - 1.0 ml of blood to fill the window, and to clear the artery of potential thromboses.
h) Bandage (either experimental fibrin sealant powder or IgG powder placebo control) placed onto the arteriotomy site, a custom made cylindrical molded platform (made with Bondo TM epoxy compound molded in a cylinder with one end against the internal side of a 400g rat's back and gently sanded to a general conformation, weight = 15.2g) covered in thin plastic wrap placed over bandage, then a 500g cylindrical brass weight (OHAUS) placed on platform and stabilized from tilting with a ring stand. This step done as quickly as possible (<1 min) and gentle manual pressure is applied throughout these manipulations.
i) Proximal aortic clamp is removed. If bleeding noted, a few seconds of manual pressure is applied and minor adjustments to position/angle of weight are made to achieve hemostasis. Often the weight tilts slightly forward less than 5 degrees. If hemostasis can not be achieved or excessive hemorrhage occurs (as indicated by MAP just prior to lifting the weight -- see exclusion criteria below), then the animal must be excluded.
j) After 10 minutes of pressure, the weight is lifted and visible hemorrhage noted. If the MAP is <60 mmHg during the one minute period prior to lifting the weight (indicating excessive hemorrhage has occured prior to this point, or that the animal is responding aberrantly to the anesthesia), then the animal must be excluded. After one more minute, the molded platform is carefully removed, exposing the undisturbed bandage.
k) The animal is monitored until death occurs from exsanguination. or if hemostasis maintained, up to a maximum of 30 minutes. A positive result is hemostasis with the animal surviving the entire 30 minutes. A negative result is hemorrhage with death by exsanguination within the 30 min. test period. An intermediate result is survival through the 30 min. test period but the MAP not maintained above 50 mmHg.
l) If the rat survives the 30 min. test period, the carotid transducer is removed and exsanguination allowed to occur. After death, the blood is swabbed from the peritoneal cavity with reweighed gauze pads and the gauze reweighed to estimate blood loss. The bandage and arteriotomy site are inspected and the arterial incision is remeasured to confirm a length of 4 mm +/- 0.5 mm.

### Summary of inclusionary/exclusionary criteria and experimental endpoints:

### 1) Inclusion criteria for rat selection:

a) HSD albino males
b) Weight between 400 and 500 g.
c) Age between 120 and 180 days

### 2) Inclusion criteria for procedural parameters:

a) MAP at least 80 mmHg average during the 5 minute period prior to laparotomy
b) HR between 300 and 500 bpm during the 5 minute period prior to laparotomy
c) MAP at least 60 mmHg average during the 1 minute period prior to lifting the weight
d) Length of aortic incision between 3.5 and 4.5 mm confirmed at end ofprocedure

### 3) Exclusion criteria for procedural parameters

a) Uncontrolled hemorrhage from a site other than the aortic arteriotomy
b) Death for some reason other than obvious hemorrhage
c) Uncorrectable surgical or procedural error
d) Any animals that do not meet the inclusion criteria above

### 4) Experimental endpoints

a) Primary endpoints
   - Hemostasis
   - Survival for 30 minutes after release of pressure on bandage
b) Secondary endpoints
   - MAP maintained above 50mmHg for the entire 30 minutes of survival
c) Strong positive result
   - meets both primary and secondary endpoints
d) Intermediate positive result
   - Meets primary but not secondary endpoints
e) Negative result
   - Hemorrhage leading to death within 30 min.
RESULTS: This study showed that with the layered bandage applied by two different techniques yielded a success rate of exactly 50% by either method with an N of ten animals included per group.

With the weight and platform method, ten of nineteen animals were included in the study (5 pre-treatment and 4 post-treatment exclusions) with two post-treatment exclusions not surviving despite apparent hemostasis. Five of the ten included animals survived the 30 minute test period, while three of these survivors exhibited complete hemostasis.

With the application of a single bandage using finger pressure, ten of twelve animals were included (1 pre-treatment, and 1 post-treatment exclusion which died despite apparent hemostasis) with five of the ten subjects surviving thirty minutes. A second bandage was applied in an additional six animals, four of which were included (both pre-treatment exclusions), and two of the four included animals survived thirty minutes. Often, there was not enough time to apply a second bandage before too much blood had been lost. In nearly all cases of failure, the blood flow from the artery dissected the plane between the bandage and the tissue -- in only one case was blood seen flowing through the bandage matrix. The adherence of the bandage to the tissues was good except where natural hematomas had formed under the bandage creating a pocket of weaker fibrin. In many cases. the bandage was not fully saturated by the blood.

### Arterial hemorrhage model in the rabbit

The rabbit aorta model has several advantages over the rat model: the artery can be isolated from the underlying tissue to provide direct access to the injury site, a plate can be placed under the aorta to provide a background for applying pressure, the artery is large enough to test various incision sizes and configurations, and the rabbit has a large enough blood volume to tolerate reasonable blood losses necessary to soak the bandage or apply multiple bandages. A disadvantage of the rabbit is lack of a good anesthetic regimen that can maintain high blood pressures -- a problem that was addressed by the use of vasopressors.

New Zealand white male rabbits weighing 2.9-3.3 kg have been used in this study. Rabbits are premedicated with buprenorphine and glycopyrrholate, and anesthesia induced with ketamine and xylazine, administered 1M. An endotracheal tube is placed via tracheotomy and mechanical ventilation assistance is provided. Anesthesia is maintained by isoflurane in oxygen. The animals' condition is monitored by electrocardiography and visual observation. A 22 gauge angiocath is inserted in the left medial ear vein and IV drip of Lactated Ringer's solution is administered at a surgical rate of 20 ml/Kg/hr. Bilateral vagotomy is performed in order to avoid vagally-mediated cardiovascular effects. A solid state pressure transducer is inserted in the right common carotid artery to monitor blood pressures and computer recording is initiated.

The abdominal aorta is exposed via a ventral midline laparotomy, and the aorta is carefully dissected away from the vena cava and surrounding tissues. Small branching arteries are ligated or cauterized, isolating a 6 - 8 cm length of artery. A plastic rectangular plate is inserted under exposed portion of the aorta. Blood flow is occluded distally and proximally with Jacobsen hemostatic forceps. A 5 mm longitudinal incision is made with a microblade and fine scissors.

A 15 mg/cm² (2.4 x 2.4 cm.) fibrinogen/thrombin/fibrinogen bandage applied to an injured site by a variety of techniques being tested in the initial studies. A soft get-filled plastic pad is applied over the bandage and 500 gram weight is placed on the top of the pad. The clamps are removed and the weight is used to apply pressure for ten minutes (this time period will vary in subsequent studies). After the specified time, the weight and gel-pad are removed. Shortly afterward the plastic plate is carefully removed from under the bandage. The observation period is thirty minutes after removal of the weight. Five minutes into the observation period, a phenylephrine IV drip is initiated to boost the animal's mean arterial pressure above 100 mmHg and is continued at increasing rates until the end of the observation period or death of the animal from exsanguination.

The preliminary model development study involves at least the following five groups: 1) dry bandage placed under the aorta, soaked with 2 - 3 mL of saline, and folded around the injury site before the weight is applied; 2) as in group 1, but the bandage is soaked with blood freshly drawn from the animal; 3) dry bandage placed flat over the incision and soaked with saline just before pressure is applied; 4) dry bandage placed flat over the incision and allowed to soak with blood flowing from the artery; and 5) a placebo bandage applied by the most effective technique above. In the fourth group, the clamps are released immediately after the bandage is in place to allow blood flow, and the weight is applied as soon as blood flow is visibly soaking the bandage.
RESULTS: Hemostasis occurs in 50% of animals when the bandage is hydrated by blood flowing from the wound itself, but hemostasis is achieved in 100% of animals under controlled conditions where saline was used to wet the bandage. The reason for the differences seen when blood flowing from the artery was used to soak the bandage are unclear.

### Prostatectomy hemorrhage model in the dog

Fibrinogen/thrombin/fibrinogen bandages were prepared for a canine prostatectomy study. The fibrinogen and thrombin were removed from the refrigerator and allowed to warm to 25°C for 2 hours. Dispo plastic molds (3.0cm x 2.4cm and 3.7cm x 2.4cm) were placed on dry ice and sprayed with 300µL of 2% sucrose. Vicryl™ was applied to the molds and pressed into place. The molds were incubated at -80°C for 1 hour.

Fibrinogen was solubilized with 11mL of 2% sucrose to a concentration of 36.4 mg/mL. Each bandage had a final fibrinogen amount of 15mg/cm². The 3.0 x 2.4cm molds received 1.5mL of fibrinogen; whereas, the 3.7 x 2.4cm molds received 1.75mL of fibrinogen. The bandages were frozen at -80°C for 1 hour.

Thrombin was solubilized with 0.5mL of 40mM CaCl₂ to a concentration of 2000U/mL. All of the bandages were placed on dry ice and sprayed with thrombin. Each bandage received thrombin at 37.5U/cm². The bandages were returned to -80°C for an additional 1 hour.

The bandages were placed on dry ice and received a second layer of fibrinogen (identical to the first layer). The bandages were returned to -80°C for 2 hours and then they were placed into the freeze-dryer.

In this prostatectomy model. a midline abdominal incision is made and a splenectomy performed. The prostate is bluntly dissected free and the urethra is isolated just distal to the prostate. The urethra is incised anteriorly, the foley catheter is drawn upward into the wound. and the posterior urethra transected. The vascular pedicles to the prostate are transected sharply. Bleeding is controlled with lap sponges and manual pressure while the bladder neck is dissected free from the prostate. The prostate is delivered from the operative field.

At this point, if there is bleeding, vessels are isolated and ligated. The time to achieve hemostasis is measured from the time the prostate is delivered until there is no further bleeding. Acute blood loss is calculated by subtracting the weight of the lap sponges preoperatively from the post operative weight.

The study was comprised of four experimental treatment groups: control, fibrin sealant bandage, placebo bandage, and liquid fibrin sealant. The control is the application of lap sponges and manual pressure. The experimental group is the application of the fibrin sealant bandage over the bleeding area prior to placing the lap sponges and manual pressure. Additionally, the anastomosis is wrapped with two ( 1 x 3 cm) bandages. The placebo bandage is a visually identical layered lyophilized IgG protein preparation which is treated the same as the experimental fibrin sealant bandage group. The liquid fibrin sealant is fibrinogen, Factor XIII, and thrombin solutions that are mixed and applied to bleeding surfaces prior to pressure application. Additionally. 6ml of this sealant is applied to the anastomosis.
RESULTS: The preliminary results from this canine prostatectomy study indicate that the fibrin sealant bandage successfully controls bleeding in this model (Table 12).

**Table 12.**

| **Efficacy of fibrin sealant bandage in canine prostatectomy study** | | | | |
|---|---|---|---|---|
| | Control | Placebo bandage | Fibrin sealant bandage | Liquid fibrin sealant |
| Number of animals | 4 | 5 | 3 | 3 |
| Blood loss (mL) | 240 ± 22 | 249 ± 22 | 114 ± 9 | 238 ± 32 |
| Ligated vessels | 4/4 | 5/5 | 0/3 | 3/3 |
| In the table, "ligated vessels" refers to the number of animals that needed vessels ligated in order to stop bleeding after the prostate is removed. Only the dogs that were treated with the fibrin sealant bandage did not need vessels ligated. | | | | |

### Liver injury hemorrhage model in the pig

Layered bandages were prepared for a swine liver injury model. Fibrinogen and thrombin were removed from the refrigerator and allowed to warm to 25°C for 2 hours. Forty-four square petri dish molds (10.1cm x 10.1cm), with a surface area of 103cm², were placed on shelf trays from the freeze-dryer. Each petri dish received 20mL of 2% sucrose to yield 194µL/cm².

After adding the 2% sucrose, the molds were placed at -80°C for 2 hours until frozen. Vicryl™ was applied and pressed into place. The molds were returned to -80°C: for 1 hour.

Each vial of fibrinogen was solubilized with 10mL of 2% sucrose to a final concentration of 16mg/mL. The molds received 25mL of fibrinogen and were returned to -80°C for 2 hours until frozen.

Thrombin was solubilized with 0.5mL of 40mM CaCl₂ to a concentration of 2000U/mL. The bandages were placed on dry ice. sprayed with thrombin and incubated at -80°C for 1 hour.

An additional 25mL of fibrinogen was added on top of the thrombin. The bandages incubated at -80°C for 2 hours until they were placed into the freeze-dryer.

After freeze-drying, the bandages were packaged and sent testing in the swine liver injury study.

Studies were conducted to test the efficacy of the fibrin sealant bandage and to determine the effect of fibrinogen dose on blood loss. Under general anesthesia, each pig underwent laparotomy and splenectomy. Following a 15 minute period, a Grade I V liver injury was induced using a specially designed instrument. After 30 seconds, the bandages were applied to the injury. Resuscitation with lactated Ringer's solution was simultaneously initiated and the animals were resuscitated to pre-injury mean arterial pressure. The abdominal incision was then closed and the pig was monitored for 60 minutes. The animals were euthanized at 60 minutes. Blood samples were collected at pre-injury and at 30 and 60 minutes post-injury. CBC, fibrinogen, PT, and PTT were determined at cach time point. At 60 minutes, the total fluid use and the volume of blood present in the abdominal cavity (total blood loss) were determined. Additionally, the liver was excised and the injury was scored to confirm a Grade IV injury.
RESULTS: The study of a porcine liver injury model to test the efficacy of the fibrin sealant (FS) bandage to prevent hemorrhage has shown that the FS bandage is far superior to standard treatment using fluid resuscitation and gauze application. The study consisted of four groups: (1) no treatment, (2) IgG bandage (placebo), (3) gauze packing, and (4) fibrin sealant bandage . All animals in each group received fluid resuscitation to compensate for the shed blood. The animals were monitored for 60 minutes.

Group 1 (no treatment group) lost 6.025 ± 1.020cc of blood per animal and the animals died. Group 2 (placebo bandage) lost 4.222 ± 1.554cc of blood per animal and the animals died. Group 3 (gauze application group) lost 1,104 ± 263cc of blood per animal. Group 4 (fibrin sealant bandage group) lost 544 ± 104cc of blood per animal.

Similar results were observed when pigs were made coagulopathic by both dilution and hypothermia, except that under these more stringent conditions, the animals treated with guaze packing exsanguinated, while those treated with the fibrinogen/thrombin/fibrinogen bandage survived.

### Initial bandage fibrinogen dosage study

The blood loss in the control group was 2819 ± 629mL (mean ±SEM) which was significantly greater than any of the treatment groups (p<.05).

Blood loss in the fibrin sealant bandage treatment groups did not differ significantly from each other. The volumes were 588 ± 629mL. 632 ± 703mL, and 758 ± 629mL in the 15, 8, and 4mg fibrinogen / cm² groups. respectively.

The total fluid resuscitation volume was 2757 ± 565mL, 1448 ± 505mL, 1775 ± 505mL, and 2208 ± 565mL for the control, 15mg, 8mg, and 4mg fibrinogen / cm² groups, respectively. The fibrinogen and all CBC components decreased as the blood loss increased. PT and PTT increased.

Some of the clots from the fibrin sealant bandages used in the pigs were examined: 3/3 control bandages did not adhere. 3/3 in the 15mg/cm² bandage group adhered well, 3/3 in the 8mg/cm² bandage group adhered, and 3/3 in the 4mg/cm² bandage group adhered slightly. One pig died prior to 60 minutes in each of the control, 4mg, and 8mg treatment groups. All of the animals survived to 60 minutes in the 15mg group. This study confirms the previous finding that the fibrin sealant bandage decreases blood loss following Grade V liver injury in swine.

The data suggest that fibrinogen levels from 4mg to 15mg/cm² are effective for hemorrhage control. Based on assessments of the clot quality, it appears that fibrinogen dose is related to quality. The higher dose is associated with more firm and tightly adhered clots. While lower fibrinogen doses are effective for hemorrhage control during the initial 60 minutes. longer term survival will likely depend on clot quality. Future survival studies will focus on determining the amount of fibrinogen that produces both short term (60 min) and longer term hemostasis and survival.

## Claims

1. A hemostatic sandwich bandage for treating wounded tissue in a patient which comprises:
(i) a first fibrinogen layer;
(ii) a thrombin layer adjacent to said first fibrinogen layer; and
(iii) a second fibrinogen layer adjacent to said thrombin layer.

2. A hemostatic sandwich bandage for treating wounded tissue in a patient which comprises:
(i) a resorbable material layer;
(ii) a first fibrinogen layer adjacent to said resorbable material layer;
(iii) a thrombin layer adjacent to said first fibrinogen layer; and
(iv) a second fibrinogen layer adjacent to said thrombin layer.

3. A hemostatic sandwich bandage for treating wounded tissue in a patient which comprises:
(i) a first fibrinogen layer;
(ii) a resorbable material layer adjacent to said first fibrinogen layer;
(iii) a thrombin layer adjacent to said resorbable material layer; and
(iv) a second fibrinogen layer adjacent to said thrombin layer.

4. The hemostatic bandage according to any one of claims 1-3, further comprising a backing material.

5. The hemostatic sandwich bandage according to any one of claims 2-3, wherein said resorbable material is selected from the group consisting of glycolic acid polymers, lactic acid polymers and glycolic acid/lactic acid co-polymers.

6. The hemostatic sandwich bandage according to any one of claims 1-3, wherein one or more of said layers contains a solubilizing agent.

7. The hemostatic sandwich bandage according to any one of claims 1-3, wherein one or more of said layers contains a filler.

8. The hemostatic sandwich bandage according to any one of claims 1-3, wherein one or more of said layers contains a binding agent.

9. A hemostatic sandwich bandage according to any one of claims 1-3 for use in a method of treating wounded tissue.

## Patentansprüche

1. Hämostatischer Sandwichverband zur Behandlung von verwundetem Gewebe bei einem Patienten, enthaltend:
(i) eine erste Fibrinogenschicht;
(ii) eine Thrombinschicht, die zu der ersten Fibrinogenschicht benachbart ist; und
(iii) eine zweite Fibrinogenschicht, die zu der Thrombinschicht benachbart ist.

2. Hämostatischer Sandwichverband zur Behandlung von verwundetem Gewebe bei einem Patienten, enthaltend:
(i) eine Schicht aus resorbierbarem Material;
(ii) eine erste Fibrinogenschicht, die zu der Schicht aus resorbierbarem Material benachbart ist;
(iii) eine Thrombinschicht, die zu der ersten Fibrinogenschicht benachbart ist; und
(iv) eine zweite Fibrinogenschicht, die zu der Thrombinschicht benachbart ist.

3. Hämostatischer Sandwichverband zur Behandlung von verwundetem Gewebe bei einem Patienten, enthaltend:
(i) eine erste Fibrinogenschicht;
(ii) eine Schicht aus resorbierbarem Material, die zu der ersten Fibrinogenschicht benachbart ist;
(iii) eine Thrombinschicht, die zu der Schicht aus resorbierbarem Material benachbart ist; und
(iv) eine zweite Fibrinogenschicht, die zu der Thrombinschicht benachbart ist.

4. Hämostatischer Verband gemäß irgendeinem der Ansprüche 1-3, der weiterhin ein Trägermaterial enthält.

5. Hämostatischer Sandwichverband gemäß irgendeinem der Ansprüche 2-3, bei dem das resorbierbare Material ausgewählt ist aus der Gruppe, bestehend aus Glycolsäurepolymeren, Milchsäurepolymeren und Glycolsäure/Milchsäure-Copolymeren.

6. Hämostatischer Sandwichverband gemäß irgendeinem der Ansprüche 1-3, bei dem eine oder mehrere der Schichten ein Lösungshilfsmittel enthält.

7. Hämostatischer Sandwichverband gemäß irgendeinem der Ansprüche 1-3, bei dem eine oder mehrere der Schichten ein Füllmaterial enthält.

8. Hämostatischer Sandwichverband gemäß irgendeinem der Ansprüche 1-3, bei dem eine oder mehrere der Schichten ein Bindemittel enthält.

9. Hämostatischer Sandwichverband gemäß irgendeinem der Ansprüche 1-3 zur Verwendung in einem Verfahren zur Behandlung von verwundetem Gewebe.

## Revendications

1. Bandage hémostatique multicouches pour le traitement d'un tissu lésé chez un patient qui comprend :
(i) une première couche de fibrinogène ;
(ii) une couche de thrombine adjacente à ladite première couche de fibrinogène ; et
(iii) une seconde couche de fibrinogène adjacente à ladite couche de thrombine.

2. Bandage hémostatique multicouches pour le traitement d'un tissu lésé chez un patient qui comprend :
(i) une couche de matériau résorbable ;
(ii) une première couche de fibrinogène adjacente à ladite couche de matériau résorbable ;
(iii) une couche de thrombine adjacente à ladite première couche de fibrinogène ; et
(iv) une seconde couche de fibrinogène adjacente à ladite couche de thrombine.

3. Bandage hémostatique multicouches pour le traitement d'un tissu lésé chez un patient qui comprend :
(i) une première couche de fibrinogène ;
(ii) une couche de matériau résorbable adjacente à ladite première couche de fibrinogène ;
(iii) une couche de thrombine adjacente à ladite couche de matériau résorbable ; et
(iv) une seconde couche de fibrinogène adjacente à ladite couche de thrombine.

4. Bandage hémostatique selon l'une quelconque des revendications 1 à 3, comprenant en outre un matériau de renforcement.

5. Bandage hémostatique multicouches selon l'une quelconque des revendications 2 à 3, dans lequel ledit matériau résorbable est choisi dans le groupe composé de polymères d'acide glycolique, de polymères d'acide lactique et de copolymères d'acide glycolique/acide. lactique.

6. Bandage hémostatique multicouches selon l'une quelconque des revendications 1 à 3, dans lequel une ou plusieurs desdites couches contien(nen)t un agent de dissolution.

7. Bandage hémostatique multicouches selon l'une quelconque des revendications 1 à 3, dans lequel une ou plusieurs desdites couches contien(nen)t un agent de remplissage.

8. Bandage hémostatique multicouches selon l'une quelconque des revendications 1 à 3, dans lequel une ou plusieurs desdites couches contien(nen)t un liant.

9. Bandage hémostatique multicouches selon l'une quelconque des revendications 1 à 3, destiné à être employé dans un procédé de traitement d'un tissu lésé.
